(19) Europäisches Patentamt
European Patent Office
Office européen des brevets

(11) Numéro de publication: 0 002 411
B1

# (12) FASCICULE DE BREVET EUROPEEN

(45) Date de publication du fascicule de brevet: 10.06.81

(21) Numéro de dépôt: 78400198.4

(22) Date de dépôt: 27.11.78

(51) Int. Cl.³: C 07 C 13/20, C 07 C 2/46, B 01 J 31/28, C 07 C 7/177

## (54) Catalyseur constitué d'un mélange de chlorure de dinitrosylfer et de bis (cyclooctadiène) nickel et son utilisation pour la dimérisation des dioléfines conjuguées.

(30) Priorité: 29.11.77 FR 7735924

(43) Date de publication de la demande: 13.06.79 Bulletin 79/12

(45) Mention de la délivrance du brevet: 10.06.81 Bulletin 81/23

(84) Etats Contractants Désignés: BE DE GB NL

(56) Documents cités: FR - A - 2 225 401

(73) Titulaire: Société Chimique des Charbonnages
Tour Aurore Place des Reflets Cédex no 5
F-92080 Paris La Defense 2 (FR)

(72) Inventeur: Petit Francis
27 rue de Flandre
F-59290 Wasquehal (FR)
Inventeur: Huchette, Dominique
18 rue de l'Eglise
F-62800 Vendin-le-Vieil (FR)
Inventeur: Yax, Emile
12 rue d'Alger
F-57600 Forbach (FR)
Inventeur: Abab, Aurélien
37 Avenue de la Blies
F-57200 Sarreguemines (FR)
Inventeur: Thery, Bernard
54 impasse St Nicolas
F-62290 Noeux-les-Mines (FR)

(74) Mandataire: Dubost, Thierry Pierre et al,
Société Chimique des Charbonnages B.P. No. 1
Service Brevets
F-62160 Bully Les Mines (FR)

EP 0 002 411 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Catalyseur constitué d'un mélange de chlorure de dinitrosylfer et de bis (cyclooctadiène) nickel et son utilisation pour la dimérisation des dioléfines conjuguées.

La présente invention a pour objet un procédé de dimérisation sélective de diènes conjugués, cette dimérisation conduisant à des composés à structure cyclohexènique. Ainsi par dimérisation du butadiène-1,3 suivant le présent procédé on obtiendra uniquement du Vinyl-4-cyclohexène-1 la dimérisation de l'isoprène conduira à un mélange de diméthyl vinylcyclohexènes et de méthyl isopropényl cyclohexènes isomères.

Un second objet de l'invention est la catalyseur utilisable pour mettre en oeuvre ledit procédé.

On a déjà décrit des procédés et catalyseurs de dimérisation sélective des diènes conjugués, et en particulier du butadiène-1,3. Ainsi le brevet japonais n° 74/47 737 montre que l'on peut dimèriser le butadiène-1,3 en vinylcyclohexène à l'aide d'un catalyseur $Fe(CO)_5$/NOCl, sélectivement et avec des rendements qui atteignent 100% en 4 h à 90°.

Le brevet français n° 1 502 141 met en oeuvre en catalyseur composé d'un halogénure de dinitrosylfer, d'un réducteur (organo-magnésien, organo-aluminique) et d'un troisième composant, qui permet de dimériser sélectivement le butadiène-1,3 en vinylcyclohexène à température ambiante; les rendements atteignent 98—99% en 5 à 8 h de réaction. Les brevets des Etats-Unis d'Amérique n° 3 655 793 et 3 767 593 mettent en jeu comme catalyseur le couple halogénure de dinitrosylferréducteur (organoaluminique, borohydrure, alumino-hydrure). Le réducteur le plus performant $[(C_2H_5)_2AlCl]$ conduit à un rendement de 96% en 16 h de réaction à 20°C. Le brevet français n° 2 225 401 décrit l'utilisation également du couple halogénure de dinitrosylfer/réducteur, le réducteur étant un métal carbonyle. L'optimum de température pour la dimérisation semble situé aux environs de 60°C.

Les procédés décrits antérieurement ne sont pas satisfaisants: soit qu'ils mettent en oeuvre des sustances extrêmement toxiques, comme les métaux carbonyles, soit qu'ils nécessitent des températures de réaction élevées et des temps de réaction longs. D'autre part, la Demanderesse a constaté expérimentalement que certains systèmes catalytiques, décrits comme donnant des résultats satisfaisants avec le butadiène-1,3, ne convenaient pas pour dimériser le butadiène-1,3 contenu dans une fraction d'hydrocarbures ("coupe C4") provenant du craquage à la vapeur d'une charge pétrolière, comme le naphta.

La demanderesse a trouvé qu'en utilisant comme catalyseur le couple chlorure de dinitrosylfer/Bis (cyclooctadiène)Nickel. ($Fe(NO)_2Cl$/N (cyclooctadiène-1,5$)_2$), on pouvait transformer sélectivement les hydrocarbures diéniques conjugués en dimères à noyau cyclohexène. Ces hydrocarbures diéniques seront, par exemple le butadiène-1,3 et l'isoprène (méthyl-2-butadiène-1,3). Ainsi la présence du ligande carbonyle est inutile pour cette dimérisation.

La demanderesse a trouvé d'autre part que ce système catalytique convenait également à la dimérisation en vinyl-4 cyclohexène-1 du butadiène-1,3 contenu dans une fraction d'hydrocarbures communément appelée coupe C4 et provenant du craquage à la vapeur d'une fraction pétrolière comme le naphta.

La demanderesse a trouvé en outre qu'il était préférable pour augmenter de façon sensible les taux de conversion, de faire subir à cette coupe C4 un traitement ayant pour but d'éliminer de ladite coupe C4 les composés acétyléniques qu'elle contient en faible proportion.

Ainsi la présente invention propose un procédé de dimérisation sélective d'hydrocarbures dioléfiniques conjugués en composés à noyau cyclohexène caractérisé en ce que l'on utilise comme catalyseur le couple chlorure de dinitrosylfer/Bis (cyclooctadiène) Nickel.

Ce procédé est secondairement caractérisé en ce qu'on utilise comme matière première pour la dimérisation du butadiène-1,3 contenu dans une coupe C4, de préférence une coupe C4 qui aura été préalablement traitée de façon à éliminer, ou transformer an hydrocarbures diéniques, les hydrocarbures acétyléniques qu'elle contient.

Ce traitement pourra consister en une hydrogénation sélective par des moyens connus en soi des hydrocarbures acétyléniques contenus dans cette fraction C4. Il pourra également consister en une mise en contact par des moyens connus en soi de la coupe C4 avec des composés capables de provoquer la formation d'acétylures métalliques que seront ensuite éliminés par tout moyen de séparation convenable (filtration, décantation etc. . .). On pourra également utiliser des moyens connus comme l'extraction liquide ou la distillation extractive.

Le chlorure de dinitrosylfer entrant dans la composition du ctalyseur selon l'invention sera préparé par tout moyen connu, en particulier par la réaction:

$$Fe + FeCl_2 \text{ anhydre} + 6NO \rightarrow 2\ Fe(NO)_3Cl \underset{\text{sublimation}}{\rightarrow} [Fe(NO)_2Cl]_2 + 2\ NO$$

ou par réaction de NO sur le mélange
$Fe + FeCl_3$ dans le THF (tétrahydrofuranne).
On considère en général que le chlorure de dinitrosylfer est sous forme dimère; dans ce que suit on

exprimera toujours les quantités et concentration de ce composé comme s'il était sous forme monomère.

Le Bis (cyclooctadiène) Nickel utilisé est un produit commercial fourni, par exemple, par la firme MERCK and Co.

Le mélange de ces produits pourra indifféremment être fait préalablement à la dimérisation, ou en présence des hydrocarbures à dimériser.

Le procédé selon l'invention sera mis en oeuvre dans une gamme de températures assez large allant par exemple de −10 à +80°C. Mais de préférence la température de dimérisation sera située entre 0 et +20°C et mieux, aux environs de +5°C.

En effet, la demanderesse a constaté, de façon surprenante, que partant de la température ambiante, les rendements augmentaient en opérant à des températures plus basses, ce qui est contraire à ce qui se passe si au lieu du Bis (cyclooctadiène) Nickel on utilise le ferpentacarbonyle $Fe(CO)_5$ comme décrit dans le brevet français 2 225 401.

Le rapport atomique

$$\frac{Fe}{Ni}$$

dans le couple catalytique pourra être compris entre 0,5 et 3,6. Mais il sera préférable qu'il soit voisin de 1.

Enfin on pourra, si on le désire, diluer le mélange réactionnel à l'aide d'un solvant. On pourra par exemple utiliser un hydrocarbure aromatique halogéné ou non, un hydrocarbure aliphatique saturé, l'éther diméthylique du diéthylène-glycol (DIGLYME). Les exemples non limitatifs suivants illustrent l'invention; dans ces exemples le temps de réaction a été souvent volontairement limité ce qui explique les taux de conversion obtenus; en allongeant la durée de la réaction on améliorerait ces taux de conversion.

Exemples 1 à 17: Dimérisation de l'isoprène.

Dans un autoclave sec de 320 ml muni de moyens de mesure, de chargement, d'entrée et de sortie de gaz, d'une double enveloppe pour fluide thermostaté, d'une agitation et d'un dispositif de sécurité, on introduit aprés purge à l'azote, le couple catalytique éventuellement du toluène, puis l'isoprène. La mise en contact du couple catalytique, du solvant éventuel et de l'isoprène se fait en dessous de 5°C. La température choisie pour la réaction n'est établie qu'après cette opération.

Les dimères obtenus à la fin de la réaction sont dosés par chromatographie gazeuse.

Les conditions opératoires et rendements obtenus sont consignés dans les tableaux I, II, et III.

Bien évidemment, dans le cas de l'isoprène il peut se former plusieurs produits mais on notera qu'ils ont tous la structure cyclohexénique. Il ne se forme pas, en particulier, de produit à structure cyclooctadiénique.

TABLEAU I

| EX | T°C | $Fe(NO)_2$-Cl-millimoles | Rapport Fe/Ni | taux de conversion % | $\frac{X_T}{L_T}$ |
|---|---|---|---|---|---|
| 1 | 13 | 1,21 | 1,23 | >97,5 | 13,7 |
| 2 | 20 | 1,10 | 1,20 | 85 | 12,9 |
| 3 | 30 | 1,10 | 1 | 82,8 | 12,3 |
| 4 | 40 | 1,19 | 1,18 | 67,9 | 10,9 |
| 5 | 60 | 1,16 | 1,07 | 54,3 | 9,5 |
| 6 | 80 | 1,07 | 1,02 | 32,5 | 8,1 |

Isoprène : 0,1 mole (10 ml)

Solvant : Toluène 20 ml

rapport molaire $\frac{\text{Isoprène}}{Fe(NO)_2Cl} \simeq 85$

Temps de séjour : 3 h

$X_T$ : somme des deux diméthyl-1,4 et 2,4 vinyl-4 cyclohexène-1

$L_T$ : somme des deux limonènes (méthyl-1 et méthyl-2 isopropényl-4 cyclohexène-1)

Il ne se forme pas de composé cyclooctadiènique, ou cyclododécatriénique, non plus que d'oligomères linéaires ou de polymères supérieurs de l'isoprène.

TABLEAU II

| EX | 7 | 8 | 9 | 10 | 11 | 12 | 13 | 14 | 15 |
|---|---|---|---|---|---|---|---|---|---|
| $\frac{Fe}{Ni}$ | 0,46 | 0,62 | 0,97 | 1,12 | 1,16 | 1,55 | 1,68 | 2,46 | 3,59 |
| Taux de conversion % | 56,8 | 77 | 90,5 | 91,1 | 85,8 | 84,2 | 81,9 | 70,7 | 50,9 |

Isoprène : 0,2 mole (sans solvant)

rapport molaire $\frac{\text{Isoprène}}{Fe(No)_2Cl} \simeq 85$

temps de séjour : 0,5 h

Température = 13–14°C

TABLEAU III

| EX | Température °C | Fe/Ni | Rapport molaire $\frac{\text{Isoprène}}{Fe(NO)_2Cl}$ | Taux de conversion % |
|---|---|---|---|---|
| 16 | 0 | 0,84 | 111 | 56,5 |
| 17 | 5 | 1 | 104 | 91,6 |

isoprène : 0,22 mole

pas de solvant

Temps de séjour : 0,5 h

Exemples 18 à 30 du tableau IV – Dimérisation du butadiène-1,3

Dans le même appareillage que ci-dessus, on introduit au plus à + 5°C

les deux composants du couple catalytique puis éventuellement le solvant et enfin le butadiène-1,3. En fin de réaction le vinyl-4-cyclohexène-1 est dosé ou séparé du milieu réactionnel par simple distillation.

Le tableau IV donne les conditions utilisées et rendements obtenus.

Le seul produit formé est le vinyl-4-cyclohexène-1 (VCH)

TABLEAU IV

| EX. | Solvant | Température °C | Rapport molaire Butadiene $Fe(NO)_2Cl$ | Rapport $\frac{Fe}{Ni}$ | Temps de séjour h | Taux de conversion du butadiène en VCH % |
|---|---|---|---|---|---|---|
| 18 | Toluène (65% du mélange en poids) | −10 | 100 | 0,7 | 0,10 mn | 35 |
| 19 | ,, | + 5 | 100 | 0,7 | 0.10 mn | 90 |
| 20 | ,, | +12 | 100 | 0,7 | 0.10 mn | 85 |
| 21 | ,, | +20 | 1000 | 0,7 | 1 | 88 |
| 22 | ,, | +20 | 2500 | 0,7 | 1 | 45 |
| 23 | ,, | +20 | 100 | 0,5 | 0.10 mn | 76 |
| 24 | ,, | +20 | 100 | 1 | 0.10 mn | 85 |
| 25 | ,, | +20 | 100 | 1,8 | 0.10 mn | 70 |
| 26 | ,, | +20 | 100 | 2,6 | 0.10 mn | 38 |
| 27 | Dimethoxy éthane (76% du mélange en poids) | + 5 | 990 | 1 | 0.30 mn | 38 |
| 28 | ,, | + 5 | 1020 | 1 | 1 | 77 |
| 29 | Vinylcyclohexène-1,4 (70% du mélange en poids) | + 5 | 500 | 0,7 | 1 | 63 |
| 30 | sans | −10 | 90 | 0,72 | 1 | 95 |
| 24 bis | Toluène (26% du mélange en poids | +20 | 460 | 1 | 2 | 100 |

Exemples 31 à 35 du tableau V —

Dimérisation du butadiène contenu dans une coupe C4 issue du vapocraquage du naptha.

Dans l'autoclavage décrit ci-dessus refroidi à −10°C on introduit le couple catalytique puis la couple C4 préalablement refroidie à −10°C.

Cette coupe C4 contient, outre des butanes et butènes, 48% environ en poids de butadiène-1,3 et 0,8% environ d'acétylénique.

Les conditions opératoires et rendements obtenus figurent dans le tableau V.

Comme dans le cas du butadiène-1,3 pur, le seul produit obtenu est le vinyl-4 cyclohexène-1 (VCH). Une simple distillation permet d'éliminer les butènes et butanes puis de recueillir le vinyl-4 cyclohexène-1 pur. On peut également dimériser la coupe C4, si on le désire, en présence de solvant.

TABLEAU V

| EX. | Température °C | Rapport molaire Butadiène / $Fe(NO)_2Cl$ | Rapport Fe / Ni | Temps de séjour h | Taux de conversion de butadiène en VCH % |
|---|---|---|---|---|---|
| 31 | -10 | 142 | 0,53 | 3 | 70 |
| 32 | -10 | 124 | 0,8 | 4 | 84 |
| 33 | -10 | 105 | 1 | 5 | 100 |
| 34 | +15 | 126 | 1 | 3 h30 | 100 |
| 35 | +15 | 100 | 1 | 2 h30 | 100 |

Dans les essais 34 et 35, la coupe C4 est diluée à 60% en poids dans du toluène.

Exemples 36 et 37 du tableau VI —
Dimérisation du butadiène contenu dans une coupe C4 préalablement traité.

Le traitement a consisté faire barbotter la coupe C4 dans une solution de chlorure cuivreux ammoniacal, à température ambiante puis à sécher le produit obtenu sur tamis moléculaire.

La coupe traitée qui ne contient plus que moins de 0,1% en poids de composés acétyléniques est soumise à la dimérisation dans l'appareillage décrit ci-dessus. Les conditions opératoires et les résultats figurent dans le tableau VI.

TABLEAU VI

| EX. | Température °C | Solvant | Rapport molaire Butadiène / $Fe(NO)_2Cl$ | Rapport Fe / Ni | Temps de séjour h | Taux de conversion du butadiène en VCH % |
|---|---|---|---|---|---|---|
| 36 | -10 | sans | 92 | 1,1 | 2 | 87 |
| 37 | + 5 | Toluène (64% en poids) | 241 | 0,84 | 1 | 100 |

## Revendications

1. Procédé de conversion sélective d'hydrocarbures à doubles liaisons conjuguées en dimères à structure cyclohexénique caractérisé en ce qu'on utilise comme catalyseur de dimérisation le couple chlorure de dinitrosylfer/bis (cyclooctadiène) Nickel.

2. Procédé selon la revendication 1, caractérisé en ce que le chlorure de dinitrosylfer est sous la forme dimère $(Fe(NO)_2Cl)_2$.

3. Procédé selon l'une des revendications 1 et 2, caractérisé en ce que la dimérisation est effectuée entre −5 et +20°C.

4. Procédé selon l'une des revendications 1, 2 et 3, caractérisé en ce que le rapport atomique

$$\frac{Fe}{Ni}$$

dans le couple catalytique est compris entre 0,5 et 3,6.

5. Procédé selon l'une des revendications 1, 2, 3 et 4, caractérisé en ce que l'hydrocarbure à doubles liaisons conjuguées est l'isoprène.

6. Procédé selon l'une des revendications 1, 2, 3 et 4, caractérisé en ce que l'hydrocarbure à doubles liaisons conjuguées est le butadiène-1,3.

7. Procédé selon l'une des revendications 1, 2, 3 et 4, caractérisé en ce que l'hydrocarbure à

doubles liaisons conjuguées est contenu dans une coupe de distillation provenant du craquage à la vapeur de fractions pétrolières.

8. Procédé selon la revendication 7, caractérisé en ce que la coupe C4 a été préalablement soumise à un traitement destiné à diminuer la concentration des hydrocarbures acétyléniques qu'elle contient.

9. Procédé selon la revendication 8, caractérisé en ce que le traitement préalable consiste en une hydrogénation sélective des triples liaisons acétyléniques.

10. Procédé selon la revendication 8, caractérisé en ce que le traitement préalable consiste à mettre la coupe C4 en contact avec des composés susceptibles de former des acétylures métalliques, puis à séparer les acétylures formés.

11. Catalyseur de dimérisation sélective des hydrocarbures à doubles liaisons conjuguées, caractérisé en ce qu'il est constitué d'un mélange de chlorure de dinitrosylfer et de bis (cyclooctadiène) Nickel.

12. Catalyseur selon la revendication 11 caractérisé en ce que le chlorure de dinitrosylfer est sous la forme dimère $(Fe(NO)_2Cl)_2$.

13. Catalyseur selon l'une des revendications 11 et 12 caractérisé en ce que le rapport atomique

$$\frac{Fe}{Ni}$$

dans le mélange de chlorure de dinitrosylfer et de bis (cyclooctadiène) Nickel est compris entre 0,5 et 3,6.

**Patentansprüche**

1. Verfahren zur selektiven Umwandlung von Kohlenwasserstoffen mit konjugierten Doppelbindungen in Dimere mit Cyclohexenstruktur, dadurch gekennzeichnet, daß als Dimerisationskatalysator ein Gemisch von Dinitrosyleisenchlorid und Biscyclooctadiennickel verwendet wird.

2. Verfahren nach Anspruch 1, dadurch gekennzeichnet, daß das Dinitrosyleisenchlorid in Form des Dimeren $(Fe(NO)_2Cl)_2$ eingesetzt wird.

3. Verfahren nach Anspruch 1 oder 2, dadurch gekennzeichnet, daß die Dimerisation zwischen $-5$ und $+20°C$ vorgenommen wird.

4. Verfahren nach einem der Ansprüche 1 bis 3, dadurch gekennzeichnet, daß ein Atomverhältnis Fe:Ni im Katalysatorgemisch von 0,5 bis 3,6 angewandt wird.

5. Verfahren nach einem der Ansprüche 1 bis 4, dadurch gekennzeichnet, daß als Kohlenwasserstoff mit konjugierten Doppelbindungen Isopren verwendet wird.

6. Verfahren nach einem der Ansprüche 1 bis 4, dadurch gekennzeichnet, daß als Kohlenwasserstoff mit konjugierten Doppelbindungen 1.3-Butadien verwendet wird.

7. Verfahren nach einem der Ansprüche 1 bis 4, dadurch gekennzeichnet, daß als Kohlenwasserstoff mit konjugierten Doppelbindungen ein aus der Dampfcrackung von Erdölfraktionen stammender Destillatschnitt verwendet wird.

8. Verfahren nach Anspruch 7, dadurch gekennzeichnet, daß ein $C_4$-Schnitt verwendet wird, der zuvor einer Behandlung zur Verringerung der Konzentration darin enthaltener acetylenischer Kohlenwasserstoffe unterzogen wurde.

9. Verfahren nach Anspruch 8, dadurch gekennzeichnet, daß die Behandlung zur Verringerung der Konzentration acetylenischer Kohlenwasserstoffe in einer selektiven Hydrierung der acetylenischen Dreifachbindungen besteht.

10. Verfahren nach Anspruch 8, dadurch gekennzeichnet, daß der $C_4$-Schnitt mit zur Bildung von Metallacetyliden befähigten Verbindungen in Kontakt gebracht wird und die gebildeten Acetylide anschließend abgetrennt werden.

11. Selektiver Dimerisationskatalysator zur selektiven Dimerisierung von Kohlenwasserstoffen mit konjugierten Doppelbindungen, dadurch gekennzeichnet, daß er aus einem Gemisch von Dinitrosyleisenchloride und Biscyclooocatadiennickel besteht.

12. Dimerisationskatalysator nach Anspruch 11, dadurch gekennzeichnet, daß das Dinitrosyleisenchlorid in Form des Dimeren $(Fe(NO)_2Cl)_2$ vorliegt.

13. Katalysator nach Anspruch 11 oder 12, dadurch gekennzeichnet, daß das Atomverhältnis Fe:Ni in Gemisch von Dinitrosyleisenchlorid und Biscyclooctadiennickel zwischen 0,5 und 3,6 liegt.

**Claims**

1. Process for the selective conversion of hydrocarbons having conjugated double bonds into dimers having a cyclohexene structure, characterised in that the couple dinitrosyliron chloride/Nickel bis(cyclooctadiene) is used as the dimerisation catalyst.

2. Process according to claim 1, characterised in that the dinitrosyliron chloride is in the dimeric form $(Fe[NO]_2Cl)_2$.

3. Process according to one of claims 1 and 2, characterised in that the dimerisation is carried out between −5 and +20°C.

4. Process according to one of claims 1, 2 and 3, characterised in that the atomic ratio

$$\frac{Fe}{Ni}$$

in the catalytic couple is comprised between 0.5 and 3.6.

5. Process according to one of claims 1, 2, 3 and 4, characterised in that the hydrocarbon having conjugated double bonds is isoprene.

6. Process according to one of claims 1, 2, 3 and 4, characterised in that the hydrocarbon having conjugated double bonds is 1,3-butadiene.

7. Process according to one of claims 1, 2, 3 and 4, characterised in that the hydrocarbon having conjugated double bonds is contained in a distillation cut emanating from the steam cracking of petroleum fractions.

8. Process according to claim 7, characterised in that the C4 cut has been previously subjected to a treatment intended to reduce the concentration of acetylenic hydrocarbons which it contains.

9. Process according to claim 8, characterised in that the previous treatment consists of selective hydrogenation of the acetylenic triple bonds.

10. Process according to claim 8, characterised in that the previous treatment consists of bringing the C4 cut into contact with compounds able to form metal acetylides, then of separating the acetylides formed.

11. Catalyst for the selective dimerisation of hydrocarbons having conjugated double bonds, characterised in that it is constituted by a mixture of dinitrosyliron chloride and Nickel bis(cyclooctadiene).

12. Catalyst according to claim 11, characterised in that the dinitrosyliron chloride is in the dimeric form $(Fe[NO]_2Cl)_2$.

13. Catalyst according to one of claims 11 and 12, characterised in that the atomic ratio

$$\frac{Fe}{Ni}$$

in the mixture of dinitrosyliron chloride and Nickel bis(cyclooctadiene) is comprised between 0.5 and 3.6.